Europäisches Patentamt

European Patent Office

Office européen des brevets

(11) Veröffentlichungsnummer: **0 304 786**
**A2**

(12) **EUROPÄISCHE PATENTANMELDUNG**

(21) Anmeldenummer: 88113328.4

(22) Anmeldetag: **17.08.88**

(51) Int. Cl.4: **A61K 39/00 , A61K 39/295**

(30) Priorität: 27.08.87 US 90272

(43) Veröffentlichungstag der Anmeldung:
01.03.89 Patentblatt 89/09

(84) Benannte Vertragsstaaten:
BE DE ES FR GB IT NL SE

(71) Anmelder: **MOBAY CORPORATION**
**Patent Department Mobay Road**
**Pittsburgh Pennsylvania 15205-9741(US)**

(72) Erfinder: **Brown, Karen K.**
**10000 N.E. Harrison Drive**
**Kansas City, MO 64155(US)**
Erfinder: **Bryant, Sharon A.**
**6727 Woodland**
**Shawnee, KS 66218(US)**

(74) Vertreter: **Müller, Gerhard, Dr. et al**
**BAYER AG Konzernverwaltung RP**
**Patentabteilung**
**D-5090 Leverkusen 1 Bayerwerk(DE)**

(54) **Intranasale Impfung von Pferden mit inaktivierten Mikroorganismen oder antigenem Material.**

(57) Pferde können immunisiert werden durch intranasale Verabreichung von antigenem Material, das die Proliferation von Antikörpern hervorruft, die die Virulenz invasiver Mikroorganismen reduzieren oder neutralisieren. Diese antigenen Stoffe könne inaktivierte ganze Organismen, Extrakte aus solchen Organismen oder rekombinante DNA oder synthetische Peptid-Antigens sein. Dieses antigene Material kann mit Adjuvantien und transdermalen Trägern kombiniert werden, oder es kann nur in sterilem Wasser transportiert werden. Die intranasale Impfung kann Teil einer Gesamt-Verordnung sein, die andere Verabreichungswege wie die intramuskuläre Injektion einschließt.

EP 0 304 786 A2

## Intranasale Impfung von Pferden mit inaktivierten Mikroorganism oder antigenem Material

Die zwei grundsätzlichen Probleme der Immunisierung gegen Krankeit durch Impfung sind die Isolierung eines Antigens, das befähigt ist, die Erzeugung schützender Antikörper hervorzurufen, d.h. solcher Antikörper, die die Fähigkeit des die Krankheit verursachenden Organismus, in das Wirtstier einzudringen, hemmen oder schmälern, und die Verabreichung dieses die Immunität hervorrufenden Antigens an das Wirtstier in einer Weise, die tatsächlich die Erzeugung der schützenden Antikörper, die Immunität oder teilweise Immunität vermitteln, stimuliert. Historisch betrafen die frühesten Bemühungen um eine Immunisierung den ganzen, die Krankheit verursachenden Organismus, der entweder getötet oder erheblich geschwächt wurde. In einigen Fällen wurde der verursachende Organismus nicht einmal aus dem Medium, in dem er erhalten wurde, abgetrennt, wobei das berühmteste Beispiel die Entwicklung des Tollwut-Virus durch Pasteur ist. In vielen Fällen verursachten diese "Ganzkultur"-Impfstoffe unerwünschte Nebenreaktionen. Dementsprechend richten sich die Bemühungen in neuerer Zeit darauf, diejenigen Moleküle der verursachenden Organismen zu isolieren, die die Erzeugung der geeigneten Antikörper hervorrufen.

Diese Reihenfolge der allgemeinen Entwicklung wurde .in der Tat im Falle der Impfstoffe gegen das Eindringen von Streptococcus equi bei Pferdern verfolgt. Der anfängliche Impfstoff umfaßte abgetötete ganze Zellen, löste jedoch unerwünschte Nebenwirkungen aus, die in der Hintergrund-Diskussion der US-PS 3 852 420 im einzelnen erörtert werden. Dieses Patent betrifft die Extraktion eines Immunität hervorrufenden Antigens durch Behandlung von Zellen dieses Organismus mit heißer Säure. Dieser Extrakt enthielt jedoch noch immer Fremdstoffe, die einige unerwünschte Nebenwirkungen wie etwa Schwellungen an der Injektionsstelle verursachten. Die US-PS 4 582 798 betrifft eine Alternativ-Technik zur Extraktion des interessierenden Antigens durch Behandlung der Zellen mit Mutanolysin und einem anionischen Detergens. Diese Technik liefert einen Extrakt, der eine noch weitere verminderte Inzidenz von Nebenreaktionen verursacht.

Die anfängliche Art und Weise der Darbeitung des antigenen Materials, sei es des ganzen Organismus oder eines Extrakts war diejenige des Einführens in das Kreislaufsystem des Wirtstieres. Tatsächlich nahm man an, daß die Entwicklung zirkulierender Antikörper, die am besten durch Einführen des antigenen Materials in das Kreislaufsystem bewerkstelligt wurde, für die Entwicklung der Immunität kritisch sei. Nachfolgend wurde gefunden, daß in einigen Fällen Immunität durch lokalisierte Einführung antigenen Materials induziert werden kann. Beispielsweise berichtet der Artikel "Protective Studies with a Group A Streptococcal M Protein II Challenge of Volunteers after Local Immunization in the Upper Respiratory Tract" von S.M. Polly, R.H. Waldman, P. High, M.K. Wittner, A. Dorfman und E.H. Fox auf den Seiten 217 bis 224 von Band 131, Nummer 3 (März 1975) von The Journal of Infectious Diseases über die Immunisierung von Menschen gegen klinische Krankheit, die durch eine Klasse von Mikroorganism hervorgerufen wurde, mittels eines das passende Antigen enthaltenden Aerosol-Sprays in die Nase und den Mund-Rachen-Raum. In einer Anschluß-Studie wurde die Antwortreaktion auf einen nachfolgenden Immunitätstest durch virulente Organismen mit derjenigen von Individuen, die mittels subkutaner Injektion geimpft worden waren, und mit derjenigen ungeimpfter Individuen verglichen. Die Aerosol-Verabreichung ergab ein Maß des Schutzes und rief eine kleinere Zahl und weniger schwerwiegende Nebenreaktionen als der Weg der parenteralen Verabreichung hervor {siehe "Protective Studies with a Group A Streptococcal M Protein Vaccine III Challenge of Volunteers after Systemic or Internal Immunization with Type 3 or Type 12 Group A Streptococcus" von R. D'Alessandri, G. Plotkin, R.M. Kluge, M.K. Wittner, F.N. Fox, A. Dorfman und R.H. Waldman in The Journal of Infectious Diseases Band 138, Nummer 6 (December 1978)}.

Im Falle des Streptococcus equi ist die Darbietung des die Immunität hervorrufenden Antigens bislang die intramuskuläre Injektion der betreffenden Pferde. Im Fall der mittels Säure oder Enzym extrahierten Antigene umfaßt der injizierte Impfstoff ein Adjuvans zur Verstärkung der Antikörper-Antwort. Man hielt diese Adjuvantien für notwendig, um eine ausreichende Antikörper-Antwort zu erhalten, um die Symptome bei einem nachfolgenden Immunitätstest mit dem virulenten Organismus zu reduzieren oder zu unterdrücken. Es wurde angenommen, daß die langsame Freisetzung des Antigen-Extrakts von dem Adjuvans in den Blutstrom des geimpften Pferdes für das Erhalten eines angemessenen Immunitätsspiegels kritisch war.

Eine neuere Studie deutete darauf hin, daß die lokal am Ort des Eindringens erzeugten Antikörper bedeutsamer sein können als Serum-Antikörper (diejenigen Antikörper, die im allgemeinen im Kreislauf-System gefunden werden) für Streptococcus equi-Infektionen beim Pferd. In "Mucosal Nasopharyngeal Immune Response of Horses to Protein Antigens of Streptococcus equi" von J.E. Galon und J.F. Timeney auf den Seiten 623 bis 628 von Band 47, Nummer 3 (März 1985) von Infection and Immunity wird berichtet, daß die Kirrelation zwischen Serum-Antikörpern und dem Feldschutz sehr gering ist und daß lokal erzeugte Antikörper Antigene erkannten, die von den Serum-Antikörpern nicht erkannt worden waren. Es wird weiter die Hypothese aufgestellt, daß die Neutralisierung dieser Antigene für das Erzielen eines Schutzes wichtig sein kann. Eine intranasale Impfung wurde bisher jedoch weder vorgeschlagen noch versucht. Vielmehr erhielten einige Ponys intramuskuläre Injektionen eines mit Aluminiumhydroxid-Adjuvans versetzten Säureextrakts von Streptococcus equi, und dann wurden diese Impflinge und ungeimpfte Kontroll-Ponys dem Immunitätstest durch intranasales Sprühen virulenter Organismen unterzogen. Sämtliche der getesteten Ponys entwickelten die Krankheit ("Drusen"), waren jedoch bei erneutem Immunitätstest resistent gegen Infektion.

Die intranasale Infektion von Pferden mit getöteten Bakterien (Bakterinen) oder antigenen Extraktion wirft spezielle Probleme auf. Es ist nicht gesichert, daß ein solcher Verabreichungsweg tatsächlich irgendeinen Grad von Immunität zu erzielen vermag. Es ist herkömmliches Wissen, daß nur ein modifizierter lebender Impfstoff wirksam auf diese Weise verabreicht werden kann, weil nur ein solches Präparat eine angemessene Antigen-Konzentration darbieten würde. Tatsächlich wird über eine gewisse Arbeit mit intranasaler Impfung mit Influenza-Virus in der US-PS 4 500 513 berichtet. Weiterhin ist auch die Verabreichung des Impfstoffs wegen der Struktur der Pferdenase mit speziellen Problemen behaftet. Der Impfstoff sollte an das tonsillare Gewebe abgegeben werden, das typischerweise einige 30 bis 35 cm (12 bis 14 Zoll) einwärts der Nasenöffnung liegt. Die normale Technik der nasalen Verabreichung ist die mittels eines zerstäubten Sprays, wie im einzelnen in den im Vorstehenden erörterten Artikeln von D'Alessandri und Polly beschrieben wird. Die Verabreichung einer passenden Dosis in eine Pferdenüster würde jedoch etwa fünf Minuten erfordern, und Pferde dulden praktisch das von solchen Zerstäubern herrührende zischende Geräusch nicht. Es ist anzunehmen, daß frühere Immunitätstests mit Pferden, wie der in dem im Vorstehenden beschriebenen Artikel von Galan mitgeteilte, in der Weise durchgeführt wurden, daß die Pferde sediert wurden, bevor sie der Einwirkung des zerstäubten Sprays ausgesetzt wurden.

Fig. 1 zeigt ein Säulendiagramm, das die Wirkungen verschiedener Impfstoff-Formulierungen auf die Gesamt-Gruppe der klinischen Indices veranschaulicht, die die symptomatische Antwortreaktion von Pferden auf den Immunitätstest mittels virulenter Organismen messen.

Fig. 2 zeigt eine graphische Darstellung der täglichen Gruppen der klinischen Indices aufgetragen über der Zeit angegeben als Zahl der Tage nach Ausführung des Immunitätstests für ungeimpfte Pferde und intranasal mit der Formulierung 1 geimpfte Pferde.

Fig. 3 zeigt eine graphische Darstellung der täglichen Gruppen der klinischen Indices aufgetragen über der Zeit angegeben als Zahl der Tage nach Ausführung des Immunitätstests für ungeimpfte Pferde und intranasal mit der Formulierung 2 geimpfte Pferde.

Fig. 4 zeigt eine graphische Darstellung der täglichen Gruppen der klinischen Indices aufgetragen über der Zeit angegeben als Zahl der Tage nach Ausführung des Immunitätstests für ungeimpfte Pferde und intramuskulär mit der Formulierung 3 und intranasal mit der Formulierung 5 geimpfte Pferde.

Fig. 5 zeigt eine graphische Darstellung der täglichen Gruppen der klinischen Indices aufgetragen über der Zeit angegeben als Zahl der Tage nach Ausführung des Immunitätstests für ungeimpfte Pferde und topisch mit der Formulierung 4 geimpfte Pferde.

Fig. 6 zeigt eine graphische Darstellung der täglichen Gruppen der klinischen Indices aufgetragen über der Zeit angegeben als Zahl der Tage nach Ausführung des Immunitätstests für ungeimpfte Pferde und intramuskulär mit der Formulierung 3 geimpfte Pferde.

Es wurde gefunden, daß Pferde gegen mikrobiologische Infektion durch Aufbringen abgetöteter Organismen oder antigener Extrakte auf ihr Nasenschleimhaut- oder Tonsillengewebe immunisiert werden können. Der Impfstoff kann zweckmäßigerweise in einen geeigneten flüssigen Träger eingearbeitet und als Flüssigkeitsstrom auf die Nasenwand in der Nachbarschaft des tonsillaren Gewebes gesprüht werden. Diese Technik ist besonders wirksam für Erkrankungen der Atemwege bei Pferden, etwa durch Streptococcus zooepidemicus, Streptococcus equisimilis und Streptococcus equi, insbesondere den letztgenannten.

Das in der vorliegenden Erfindung eingesetzte antigene Material können inaktivierte ganze Organismen oder aus solchen Organismen hergestellte Extrakte (Untereinheiten), von rekombinanter DNA abgeleitetes Antigen oder synthetisches Peptid-Antigen sein. Das antigene Material leitet sich vorzugsweise von einem Bakterium ab, das Erkrankungen der Atemwege beim Pferd hervorruft, besonders bevorzugt von einem Streptococcus-Organismus der Gruppe C wie Streptococcus zooepidemicus und Streptococcus equi und am meisten bevorzugt von dem letzteren. Es kann jedoch auch ein abgetötetes Virus sein, etwa Mikroorganismen, die Pferde-Influenza oder andere Erkrankungen verusachen. So kann das antigene Material sich von jedem Organismus ableiten, dessen Virulenz in Pferden durch Antikörper des sekretorischen Systems des Pferdes neutralisiert wird. Es kann auch eine Kombination sein, etwa das abgetötete Virus der Miami- und Pennsylvania-Stämme der Pferde-Influenza mit einem Antigen-Extrakt (Enzym-Extrakt, Extrakt mit heißer Säure oder einem anderen) von Streptococcus equi.

Das antigene Material ist vorzugsweise ein Extrakt, der antigene Determinanten enthält, die die

3

Proliferation neutralisierender Antikörper auslösen. Im Fall von Streptococcus equi lehrt die US-PS 3 852 420 eine geeignete Technik der Säure-Extraktion, und die US-PS 4 582 798 lehrt eine geeignete Technik der Enzym-Extraktion. Die letztere Technik kann auch auf Kulturen von Streptococcus zooepidemicus angewandt werden.

Die vorliegende Technik der Verabreichung erlaubt die Nutzung antigener Materialien, die bei anderen Wegen der Verabreichung zu beanstandende Reaktionsfähigkeit zeigen. Beispielsweise ist bekannt, daß die Streptococcen-Organismen eine hohe Affinität zu Hautgewebe haben und mit abgetöteten Zellen oder manchen Extrakten hergestellte Impstoffe Schwellungen und andere Reizungen an der Injektionsstelle hervorrufen. Eine ähnliche Reaktionsfähigkeit wird bei intranasaler Verabreichung nicht beobachtet. Suspensionen abgetöteter ganzer Zellen von Streptococcus equi, die sowohl in Todd-Hewitt-Brühe als auch in chemisch definierten, proteinfreien Medien kultiviert worden waren, wurden nasal verabreicht, ohne daß sie irgendeine bermerkbare Reaktion hervorriefen. In der US-PS 3 852 420 war berichtet worden, daß das frühere Material bei parenteraler Injektion erhebliche Reaktionen gibt.

Die bevorzugte Technik zur Herstellung von antigenem Material, das gegen Streptococcus equi wirksam ist, ist die der Enzym-Extraktion, etwa mit Pepsin. Besonders bevorzugt wird die Verwendung eines bakteriolytischen Enzyms wie Lysozym oder Mutanolysin. Besonders bevorzugt wird die Verwendung von Mutanolysin. Es wird ebenfalls bevorzugt, der Enzym-Extraktion eine Extraktion mit einem anionischen Detergens wie Natriumdodecylsulfat folgen zu lassen. Es ist besonders vorteilhaft, den zu extrahierenden Streptococcus equi in einem proteinfreien chemisch definierten Medium zu kultivieren, etwa demjenigen, das in dem Artikel von I. van Rijn auf den Seiten 444 bis 448 in Band 27 (1980) von Infection and Immunity beschrieben ist.

Das antigene Material kann für die Anwendung in mannigfacher Weise formuliert werden. Es kann einfach in sterilem Wasser oder Trägern oder Eindickungsmitteln dispergiert oder aufgelöst werden, und Hilfsstoffe können dem Wasser zugesetzt werden. Eine zu bevorzugende Formulierung umfaßt Eindickungsmittel, insbesondere solche, die sich von Cellulose ableiten, wie semi synthetische Cellulose-Derivate einschließlich Carboxymethylcellulose, Hydroxypropylcellulose und ganz besonders Hydroxyethylcellulose. Diese Eindickungsmittel können Viskositäten, als Lösungen von 2 Gew.-% in Wasser, zwischen etwa 10 und 100 000 mPa·s (cP), vorzugsweise zwischen etwa 100 und 10 000 mPa·s (cP) und ganz besonders bevorzugt zwischen etwa 1 000 und 8 000 mPa·s (cP), haben. Bei Hydroxyethylcellulose bedingt dies aus der Grenzviskosität (Intrinsic Viscosity) bestimmte Molukulargewichte zwischen etwa 80 000 und 1 200 000. Besonders bevorzugt wird die Verwendung einer Hydroxyethylcellulose, die mit etwa 2,5 mol Ethylenoxid auf 1 mol der Anhydroglucose-Einheit ethoxyliert ist. Solche Eindickungsmittel liegen vorzugsweise in Mengen zwischen etwa 0,25 und 1 Gew.-%, bezogen auf die gesamte Formulierung, vor. Die Haut durchdringende Mittel wie Dimethylsulfoxid (DMSO) und Methylsalicylat sind ebenfalls bevorzugt enthalten. Besonders vorteilhafte Mengen liegen zwischen etwa 0,5 und 10 Gew.-%, bezogen auf die gesamte Formulierung, je nach dem angewandten speziellen, die Haut durchdringenden Mittel. Beispielsweise liegt bei DMSO der bevorzugte Bereich zwischen etwa 2,5 und 10 Gew.-%, während bei Methylsalicylat der bevorzugte Bereich zwischen 1 und 4 Gew.-% liegt. Schließlich kann die Formulierung ein anerkanntes Impfstoff-Adjuvans enthalten, etwa Aluminiumhydroxid-Gel oder diejenigen, die in der US-PS 3 919 411 gelehrt werden, vorzugsweise als Gehalte zwischen etwa 5 und 40 Vol.-%, wobei Gehalte zwischen etwa 10 und 40 Vol.-% für den erstgenannten Typ und Gehalte zwischen etwa 5 und 20 Vol.-% für den letztgenannten Typ besonders bevorzugt werden. Ein besonders bevorzugtes Adjuvans basiert auf mit Polyallylsucrose vernetzter Polyacrylsäure, das als Carbopol 934P vertrieben wird, kombiniert mit Poly oxyethylensorbitanmonooleat und Sorbitanmonolaurat, und wird vorzugsweise in Mengen zwischen 7,5 und 15 Vol.-%, bezogen auf das Gesamt-Volumen der Lösung, eingesetzt.

Der Antigen-Gehalt wird am besten durch den Effekt, den er hervorruft, definiert. Naturgemäß sollte das Antigen in Mengen vorhanden sein, die für die Erzeugung meßbarer Mengen des schützenden Antikörpers ausreichen. Ein geeigneter Test auf die biologische Aktivität von Streptococcen-Organismen ist in der US-PS 4 527 581 angegeben und betrifft die Fähigkeit des dem Test unterzogenen Materials, einem bekannten positiven Antiserum seinen Antikörper zu entziehen. Das Ergebnis wird angegeben als negativer Logarithmus der $LD_{50}$ (letale Dosis, 50 %) für mit virulenten Organismen behandelte Mäuse, die vorher mit einem bekannten Antiserum behandelt wurden, das zuvor selbst mit verschiedenen Verdünnungen des zu bewertenden antigenen Materials behandelt worden war. Demgemäß spiegelt ein hoher Wert einen hohen Gehalt an antigenem Material wieder, das die antikörper in dem bekannten Antiserum gebunden hat und damit den Effekt des Antiserums auf den virulenten Organismus, der die letale Dosis klein macht, mindert oder unterbindet. Vorzugsweise liegt das in der fertigen Formulierung vorhandene antigene Material in einer Menge vor, die ausreicht, um im Vergleich zu dem Ergebnis, das für den mit dem unbehandelten Antiserum behandelten virulenten Organismus erhalten wurde, den negativen Logarithmus der $LD_{50}$ um wenigstens 1,

vorzugsweise 1,4, zu erhöhen. Die für die Antiserum-Kontrolle und geeignetes Impfstoff-Material erhaltenen Absolut-Werte hängen naturgemäß von dem virulenten Organismus und den gewählten Antiserum-Standards ab.

Die Impfstoff-Formulierung kann auf das Lymphgewebe der Nase in jeder geeigneten Weise aufgebracht werden. Vorzugsweise wird sie jedoch als Flüssigkeitsstrom oder in Form von Flüssigkeitströpfchen auf die Wand des Nasengangs aufgebracht. Dies kann vorteilhaft mit Hilfe eines Röhrchens von kleinem Durchmesser erfolgen, das an seinem distalen Ende verschlossen ist und mehrere Löcher in der Seitenwand nahe dem verschlossenen Ende aufweist und an seinem proximalen Ende aus einer Dosierungsvorrichtung, etwa einer Injektionsspritze, gespeist wird. Das Einführen dieses Röhrchens in die Nüster des Pferdes in einer Tiefe von 15 bis 25 cm (6 bis 10 Zoll) bringt die Impfstoff-Formulierung zum richtigen Ort, ohne daß zu befürchten ist, daß das kritische Gewebe umgangen wird. Ein besonders geeignetes Röhrchen hat einen Durchmesser von etwa 1 mm und etwa oder vier Löcher von 0,5 mm in seinem Umfang in der Nähe seines verschlossenen Endes. Das Hindurchdrücken der Flüssigkeit durch solche Löcher macht den Flüssigkeitsstrom oder die großen Tröpfchen in der Praxis der vorliegenden Erfindung besonders nützlich. Eine solche Technik ermöglicht eine problemlose Feld-Behandlung ohne die Notwendigkeit besonderer Vorkehrungen oder einer Sedierung des zu behandelnden Pferdes. Typischerweise wird der Kopf des Pferdes nach hinten geneigt, so daß die Flüssigkeit an der Nasenwand zu dem Tonsillen-Gewebe hinablaufen kann.

Die Impstoff-Formulierung kann in einer beliebigen, geeigneten Dosis verabreicht werden, so lange eine ausreichende Menge des antigenen Materials zur Einwirkung gebracht wird (die zur Erhöhung des negativen Logarithmus der $LD_{50}$ in dem Vorstehenden beschriebenen Test genügt). Bevorzugt wird jedoch eine solche Konzentration des antigenen Materials, daß eine Dosis zwischen etwa 1 und 4 ml verwendet werden kann.

Der intranasale Verabreichungsweg kann für sich allein oder in Kombination mit anderen Techniken benutzt werden. Eine besonders interessante Verordnung umfaßt eine intramuskuläre Injektion und darauf folgend eine oder mehrere intranasale Verabreichungen. Für beide Verabreichungswege kann dieselbe Formulierung benutzt werden, oder für jeden Weg werden getrennte Formulierungen angewandt, z.B. eine ein Adjuvans enthaltende Formulierung für die Injektion und eine einfache Formulierung in sterilem Wasser für den nasalen Weg.

Der adäquate Schutz kann mehr als eine Verabreichung des Impfstoffs erfordern. Die Anzahl und die Zeitfolge der Verabreichungen, die für eine gegebene Formulierung optimal ist, ist vom Fachmann leicht durch Routineversuche zu ermitteln. Im Fall der Streptococcen-Organismen der Gruppe C ist ein Intervall zwischen zwei und drei Verabreichungen geeignet, das im Bereich zwischen etwa 7 und 28 Tagen liegt. Im Fall von Streptococcus equi wurde gefunden, daß eine Behandlung mit drei Dosen in Abständen von drei Wochen besonders geeignet ist, vor allem dann, wenn die erste Dosis eine intramuskuläre Injektion ist und die zwei Auffrischungsdosen intranasal verabreicht werden.

Die Erfindung wird durch die folgenden Beispiele weiter erläutert, die jedoch nicht als Einschränkung zu verstehen sind und in denen sämtliche Angaben von "Teilen" und "Prozenten" sich auf das Gewicht beziehen, sofern nichts anderes angegeben ist.

BEISPIELE

## BEISPIEL 1

Ein Impfstoff wurde aus einer Vier-Stunden-Kultur von Streptococcus equi hergestellt, die in Todd-Hewitt-Brühe bei 37 °C und einem pH von 7,2 kultiviert worden war. Die Zellen wurden von dem Wachstumsmedium abgetrennt, mit einer wäßrigen Lösung von 0,85 Gew.-% NaCl gewaschen und in einer wäßrigen Lösung von 0,85 Gew.-% NaCl resuspendiert. Diese Zell-Suspension wurde 15 min bei 121 °C und 1,03 bar Überdruck (15 psi) autoklaviert. Anschließend wurden 10 Vol.-% eines Adjuvans auf der Basis von mit Polyallylsucrose vernetzter Polyacrylsäure (im Handel erhältlich als Carbopol 934P), kombiniert mit einer Mischung aus Polyoxyethylensorbitanmonooleat, Sorbitanmonolaurat, Baumwollsamenöl und Wasser, wie sie in der US-PS 3 919 411 gelehrt wird, der Formulierung zugesetzt.

Dieser Impfstoff wurde eingesetzt, um zwei Pferde intramuskulär und drei Pferde intranasal zu immunisieren. Zwei Verabreichungen einer Dosis von 2 ml im Abstand von vier Wochen wurden für beide

5

Wege angewandt.

Diese fünf Pferde und ein Kontroll-Pferd wurden drei Wochen nach der zweiten Impfstoff-Gabe intranasal dem Immunitätstest durch Verabreichung von 10 ml einer Kultur von virulentem Streptococcus equi in der logarithmischen Phase (etwa $10^8$ Organism in 1 ml) unterworfen. Die intranasal geimpften Pferde zeigten eine 56-proz. Verringerung der klinischen Symptome, und die intramuskulär geimpften Pferde zeigten eine 46-proz. Verringerung der klinischen Symptome, verglichen mit dem Kontroll-Pferd.

Die Sera der auf beiden Wegen geimpften Pferde zeigten eine neutralisierende Wirkung auf virulenten Streptococcus equi. Insbesondere erniedrigten sie den negativen Logarithmus (oder erhöhten den Absolutwert) der $LD_{50}$ für Mäuse, die mit den verschiedenen Verdünnungen der virulenten Organismen, die mit den Sera der geimpften Pferde gemäß der Beschreibung in der US-PS 4 529 582 vorbehandelt worden waren, behandelt worden waren, wie folgt:

| Impfweg | Negativer Logarithmus der $LD_{50}$ | | |
|---|---|---|---|
| | bei der Impfung | 4 Wochen nach der Impfung | 7 Wochen nach der Impfung |
| intramuskulär | 6,5 | 4,5 | 3,9 |
| intranasal | 6,3 | 5,7 | 5,1 |

## BEISPIEL 2

Ein Impfstoff wurde aus einer Fünf-Stunden-Kultur von Streptococcus equi hergestellt, die in dem chemisch definierten Medium (CDM), das in dem Artikel von I. van Rijn auf den Seiten 444 bis 448 in Band 27 (1980) von Infection and Immunity beschrieben ist, bei 37 °C kultiviert worden war. Die Zellen wurden von dem Wachstumsmedium abgetrennt, mit einer wäßrigen Lösung von 0,85 Gew.-% Natriumchlorid gewaschen und in einer wäßrigen Lösung von 0,85 Gew.-% Natriumchlorid resuspendiert. Diese Suspension wurde 60 min auf 65 °C erhitzt. Ein Teil wurde danach mit einer ausreichenden Menge des in Beispiel 1 beschriebenen Adjuvans vermischt, so daß ein Adjuvans-Gehalt von 10 Vol.-% erhalten wurde. Ein anderer Teil wurde mit sterilem gepufferten Wasser auf 1 Teil Antigen-Quelle zu 25 Teilen Wasser verdünnt, und dies verdünnte Formulierung wurde mit einer ausreichenden Menge des in Beispiel 1 beschriebenen Adjuvans vermischt, so daß ein abschließender Adjuvans-Gehalt von 10 Vol.-% erhalten wurde.

Drei Pferde wurden intramuskulär (IM) und vier Pferde wurden intranasal (IN) mit dem Serum der vollen Stärke geimpft, und vier Pferde wurden intramuskulär mit dem averdünnten Serum geimpft. Die intramuskulären Verabreichungen erfolgten zweimal im Abstand von vier Wochen, während die intranasalen Verabreichungen viermal, in Abständen von drei Wochen, vier Wochen und sieben Wochen nach der ersten Impfung, vorgenommen wurden. Die Dosis betrug für beide Verabreichungswege 2 ml.

Diese elf Pferde und ein Kontroll-Pferd wurden etwa zehn Wochen nach der ersten Impfung intranasal dem Immunitätstest durch Verabreichung von 10 ml einer Kultur von virulentem Streptococcus equi in der logarithmischen Phase (etwa $10^8$ Organismen in 1 ml) unterworfen. Die intranasale und die intramuskuläre Verabreichung des Impfstoffs der vollen Stärke ergaben eine etwa 40-proz. Verringerung der klinischen Symptome, verglichen mit dem Kontroll-Pferd, während die intramuskuläre Verabreichung des verdünnten Impfstoffs eine 20-proz. Verringerung zur Folge hatte.

Die Sera sämtlicher geimpften Pferde zeigten eine gewisse neutralisierende Wirkung auf virulenten Streptococcus equi, gemessen mittels der Erhöhung der $LD_{50}$ für Mäuse gemäß der US-PS 4 529 582.

Die Werte des negativen Logarithmus der $LD_{50}$ für diese Behandlungen waren wie folgt:

| Impfstoff und Weg der Verabreichung | Negativer Logarithmus der $LD_{50}$ verschiedene Wochen nach Impfung | | | | | |
|---|---|---|---|---|---|---|
| | 0 | 2 | 4 | 6 | 7 | 10 |
| Unverdünnt, IM | 6,1 | 5,7 | 5,8 | 5,7 | 5,8 | 4,4 |
| Unverdünnt, IN | 6,9 | 6,7 | 6,3 | 6,3 | 6,7 | 6,1 |
| Verdünnt, IM | 5,9 | 5,4 | 5,5 | 5,0 | 5,6 | 5,5 |

Somit ergibt der intranasale Weg eine symptomatische Wirkung, die derjenigen der intramuskulären Verabreichung des unverdünnten Impfstoffs äquivalent ist, jedoch eine serologische Wirkung, die eher derjenigen der intramuskulären Verabreichung der Verdünnung 1:25 angepaßt ist. Dies gibt einen gewissen Hinweis darauf, daß die serologische Wirkung nicht immer direkt mit dem Schutz korrelieren muß.

BEISPIEL 3

A. Impfstoff-Herstellung

Eine Partie eines mittels immunstimulierendem Enzym/anionischem Detergens gewonnenen Extrakts einer Kultur von Streptococcus equi, die in dem gleichen chemisch definierten Medium wie in Beispiel 2 kultiviert worden war, wurde gemäß den Lehren der US-PS 4 582 798 hergestellt. Die Potenz dieses Materials wurde gemäß den Lehren der US-PS 4 529 581 bewertet, und es wurde gefunden, daß sie innerhalb des mit Feld-Schutz korrelierenden Bereichs lag. Im einzelnen wurde der Extrakt durch aufeinanderfolgende 16-stündige Behandlung mit 5 Einheiten Mutanolysin pro 1 ml der Original-Kultur bei 37 °C und anschließende 0,5 stündige Behandlung mit 0,05 Gew.-% Natriumdodecylsulfat hergestellt, wonach ein Präparat mit einer kombinieren den Potenz von 7,0 oder mehr erhalten wurde, gemessen als negativer Logarithmus der $LD_{50}$ bei Mäusen bei Behandlung mit einem virulenten Organismus, der mit dem Antiserum eines genesenden Pferdes behandelt worden war, wobei das Antiserum mit dem vorliegenden Präparat vorbehandelt worden war. Dieses Präparat wurde als Basis für die nachstehenden fünf Formulierungen eingesetzt:

1) 0,5 Gew.-% einer Hydroxyethylcellulose (HEC), die mit etwa 2,5 mol Ethylenoxid auf 1 mol Anhydroglucose ethoxyliert worden war, mit einer Viskosität zwischen 4 500 und 6 500 mPa•s als wäßrige Lösung von 2 Gew.-% und 2 Vol.-% Methylsalicylat wurden zu dem Extrakt-Präparat hinzugefügt.

2) 0,5 Gew.-% derselben HEC und 5,0 Vol.-% Dimethylsulfoxid wurden zu dem Extrakt-Präparat hinzugefügt.

3) 0,5 Gew.-% derselben HEC und 2,0 Gew.-% Natriumglycocholat wurden zu dem Extrakt-Präparat hinzugefügt.

4) Eine ausreichende Menge des in Beispiel 1 beschriebenen Adjuvans wurde zu dem Extrakt-Präparat hinzugefügt, damit es 10 Vol.-% der fertigen Formulierung ausmachte.

5) Das unverdünnte Extrakt-Präparat.

B. Tier-Selektion

53 Pferde gemischter Geschlechter und Rassen, die auf drei örtlichen Farmen untergebracht waren, wurden in dem Test eingesetzt. Mit den Pferden wurde eine Reihenuntersuchung auf Seronegativität mittels des (in der US-PS 4 529 582 beschriebenen) passiven Mäuse-Schutz-Tests (Passive Mouse Pro tection Assay) durchgeführt. Es war nicht möglich, insgesamt seronegative Tiere zu erhalten, und die Pferde wurden für die Impfung/den Immunitätstest nach zwei Kriterien in sechs Gruppen eingeteilt:

(1) Die mittleren Titer jeder der sechs Gruppen von Pferden sollten annähernd gleich sein. Nach der Zuordnung der Tiere lagen die mittleren Titer der verschiedenen Gruppen im Bereich von 5,9 bis 6,1.

(2) Jede der sechs Gruppen von Pferden sollte eine repräsentative Zahl von Pferden von allen drei Farmen enthalten.

Die Tiere wurden auf den jeweiligen repräsentierten Farmen geimpft. Zum Zeitpunkt des Immunitätstests wurden die Tiere auf eine Forschungs-Farm überführt, wo sie intranasal mit virulentem S. equi geimpft wurden. Auf Bitte der Eigentümer wurden nach dem Immunitätstest die Pferde von den drei jeweiligen Farmen nicht vermischt.

7

## C. Impf-Vorschrift

Fünf Gruppen von Pferden (n = 8 bis 9) wurden zwei- oder dreimal mit 2,0 ml Dosen intranasaler, intramuskulärer oder topischer Formulierungen von Bakterien-Extrakt von S. equi geimpft. Eine letzte Gruppe von Pferden wurde als Gruppe ungeimpfter Kontroll-Tiere gehalten. Die verschiedenen Impfstoffe und Impf-Vorschriften sind in Tabelle 1 zusammengefaßt.

## D. Verfahren des Immunitätstests

2 oder 3 Tage vor dem Immuniätstest (12 bis 14 Tage nach der Auffrischungsimpfung) wurden sämtliche Pferde für den Immuniätstest auf die Forschungs-Farm verbracht. Die Pferde wurden für den Immunitätstest intranasal mit einer S. equi-Kultur im logarithmischen Stadium infiziert. Etwa $10^7$ Organismen wurden jedem Pferd mit Hilfe eines modifizierten 25 cm (10 Zoll)-Katzen-Katheters verabreicht. Dieses Katheter hatte eine innere Bohrung von etwa 1 mm, war an seinem distalen Ende verschlossen und enthielt 3 oder 4 radiale Auslaßöffnungen in der Nähe dieses distalen Endes. Die Pferde wurden nach dem Immunitätstest 45 Tage lang jeden zweiten Tag einzeln untersucht.

## E. Untersuchung der Antwort auf den Immunitätstest

Wie bereits oben angegeben wurde, wurden an einzeln für den Immunitätstest infizierten Pferden die Untersuchungen während einer Beobachtungszeit von 45 Tagen nach dem Immunitätstest durchgeführt. Bei jeder Untersuchung wurden die rektalen Temperaturen aufgezeichnet, Blutproben wurden für die Zählung der weißen Blutkörperchen entnommen, und die Pferde wurden auf klinische Anzeichen wie Nasenausfluß, Abszeßbildung, Depression und Appetitlosigkeit untersucht.

Die Antwortreaktion der Pferde auf den Immunitätstest wurde mit Hilfe des in Tabelle 2 angegebenen klinischen Index gemessen. Tägliche klinische Punktwerte (DCSs) wurden jedem getesteten Pferd an jedem Beobachtungstag für vier verschiedenen Parameter zugeordnet: Abszeßbildung ($DCS_A$), Nasenausfluß ($DCS_N$), Temperaturreaktion ($DCS_T$) und Erhöhung der Zahl der weißen Blutkörperchen ($DCS_W$).

Zur Analyse der Gruppen-Antwort progessiv während der auf den Immunitätstest folgen Beobachtungsperiode wurden tägliche klinische Gruppen-Indices (DGCIs) berechnet. Der Index maß die Gruppen-Antwortreaktion an einzelnen Tagen während der 45-tägigen Beobachtungsperiode. Der DGCI für einen gegebenen Tag nach dem Immunitätstest wurde durch Summation von $DCS_A$, $DCS_N$, $DCS_T$ und $DCS_W$ für alle Pferde in einer Gruppe und Division durch die Zahl der Pferde in der Gruppe erhalten.

$$DGCI = \frac{\Sigma \quad \frac{DCS_A + DCS_N + DCS_T + DCS_W}{\text{für alle Pferde in einer Gruppe}}}{\text{Zahl der Pferde in der Gruppe}}$$

Kumulative klinische Punktwerte (CCSs) wurden an individuellen Pferdern für jeden der vier Parameter ($CCS_A$, $CCS_N$, $CCS_T$, $CCS_W$) durch Summation der täglichen Punktwerte für die jeweiligen Parameter während der 45-tägigen Beobachtungsperiode erhalten. Die allgemeine Formel für die Berechnung der CCS-Werte ist folgende:

$CCS_x = \Sigma DCS_x$ für 45 Tage nach dem Immunitätstest (worin x = A, N, T oder W).

Individuelle klinische Indices (ICIs) für jedes getestete Pferd durch Summation über $CCS_A$, $CCS_N$, $CCS_T$ und $CCS_W$ erhalten:

$ICI = CCS_A + CCS_N + CCS_T + CCS_W$

Der ICI bildet ein Maß für die Antwortreaktion des einzelnen Pferdes auf die Infektion im Immunitätstest über die gesamte Beobachtungsperiode von 45 Tagen.

Die täglichen klinischen Punktwerte wurden auch zur Analyse von Daten auf der Basis der Gruppen herangezogen. Spezifische klinische Gruppen-Indices (SGCIs) für Abszeßbildung ($SGCI_A$), Nasenausfluß ($SGCI_N$), Temperaturreaktion ($SGCI_T$) und Erhöhung der Zahl der weißen Blutkörperchen ($SGCI_W$) wurden erhalten durch Summation von $CCS_A$, $CCS_N$, $CCS_T$ oder $CCS_W$ für alle Pferde in einer Gruppe und Division durch die Zahl der Pferde in der Gruppe. Die allgemeine Formel für die Berechnung der SGCI-Werte ist wie folgt:

$$SGCI_x = \frac{\Sigma\ CCS_x\ \underline{\text{für alle Pferde in einer Gruppe}}}{\text{Zahl der Pferde in der Gruppe}}$$

Die spezifischen klinischen Gruppen-Indices liefern ein Maß für die Antwortreaktion der Gruppe auf die Infektion im Immunitätstest in bezug auf Abszeßbildung, Nasenausfluß, Temperaturreaktion oder Erhöhung der Zahl der weißen Blutkörperchen.

Schließlich wurde ein klinischer Gesamt-Gruppen-Index (TGCI), der für die Gesamt-Antwortreaktion der Gruppe auf die Infektion im Immunitätstest repräsentativ ist, durch Summation von $SGCI_A$, $SGCI_N$, $SGCI_T$ und $SGCI_W$ berechnet:

$TGCI = SGCI_A + SGCI_N + SGCI_T + SGCI_W$

## F. Serologische Prüfung

Sera wurden in der Untersuchung von allen Pferden vor ihrer Zuordnung zu den Gruppen, vor jeder Impfung und erneut vor der Infektion für den Immunitätstest entnommen. Die Sera wurden mit Hilfe des in der US-PS 4 529 582 beschriebenen passiven Mäuse-Schutz-Tests geprüft.

Tabelle 1

| Impfung mit S. equi/Immunitätstest: Impfprotokoll | | | | | | |
|---|---|---|---|---|---|---|
| Gruppe | Produkt | Anwendung | Formulierung | Dosis ml | Verordnung | Zahl der Pferde n |
| A | S. equi Bakterien-Extrakt | intranasal | 2,0 % Methylsalicylat, 0,5 % Hydroxyethylcellulose | 2,0 | 3 Dosen 3 Wochen Abstand | 8 |
| B | S. equi Bakterien-Extrakt | intranasal | 5,0 % DMSO 0,5 % Hydroxyethylcellulose | 2,0 | 3 Dosen 3 Wochen Abstand | 9 |
| C | S. equi Bakterien-Extrakt | (1) intramuskulär (2) intranasal | (1) 10 % Adjuvans aus Beispiel 1 (2) keine Additive | 2,0 2,0 | 3 Dosen 3 Wochen Abstand | 9 |
| D | S. equi Bakterien-Extrakt | topisch | 2,0 % Natriumglycocholat, 0,5 % Hydroxyethylcellulose | 2,0 | 3 Dosen 3 Wochen Abstand | 9 |
| E | S. equi Bakterien-Extrakt | intramuskulär | 10 % Adjuvans aus Beispiel 1 | 2,0 | 2 Dosen 3 Wochen Abstand | 9 |
| F | Ungeimpfte Kontrollen | entfällt | entfällt | entfällt | entfällt | 9 |

Tabelle 2

| Klinischer Index für Streptococcus equi | |
| --- | --- |
| Klinisches Zeichen | Punktzahl |
| * Abszeß-Bildung | 20 |
| ** Abszeß-Dauer | 20 |
| Rektale Temperaturen °C (°F) | |
| <38,9 (<102,0) | 0 |
| 38,9-39,1 (102,0-102,4) | 1 |
| 39,2-39,9 (102,5-103,9) | 2 |
| ≥40,0 (≥104,0) | 5 |
| Zählung der weißen Blutkörperchen | |
| >50 % Zunahme | 0 |
| 50 - 99 % Zunahme | 2 |
| >100 % Zunahme | 5 |
| Nasen-Ausfluß | |
| kein Ausfluß bis geringer Ausfluß | 0 |
| mäßiger Ausfluß | 5 |
| starker Ausfluß | 10 |

* Die Abszeß-Bildung bezieht sich auf den ersten Tag, an dem der Abszeß sichtbar wird.

** Die Abszeß-Dauer bezieht sich auf alle nachfolgenden Tage, an denen die Abszesse vor dem Abfließen sichtbar sind.

G. Ergebnisse

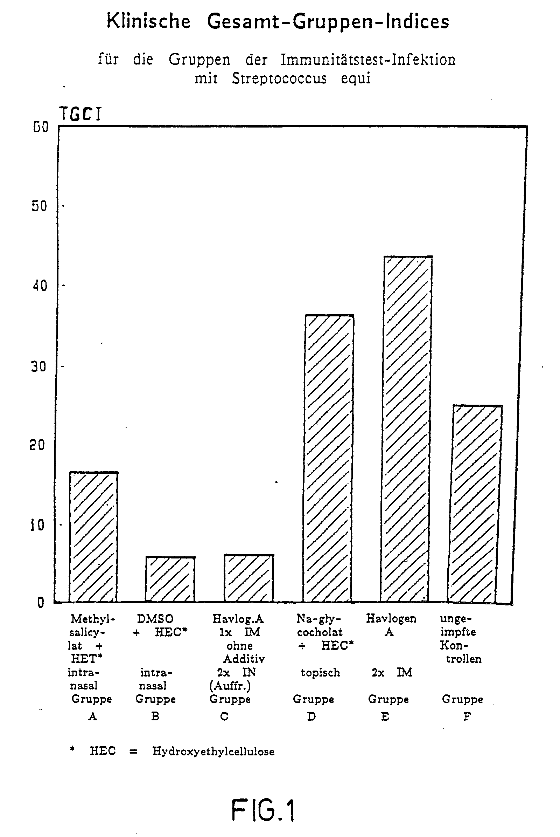

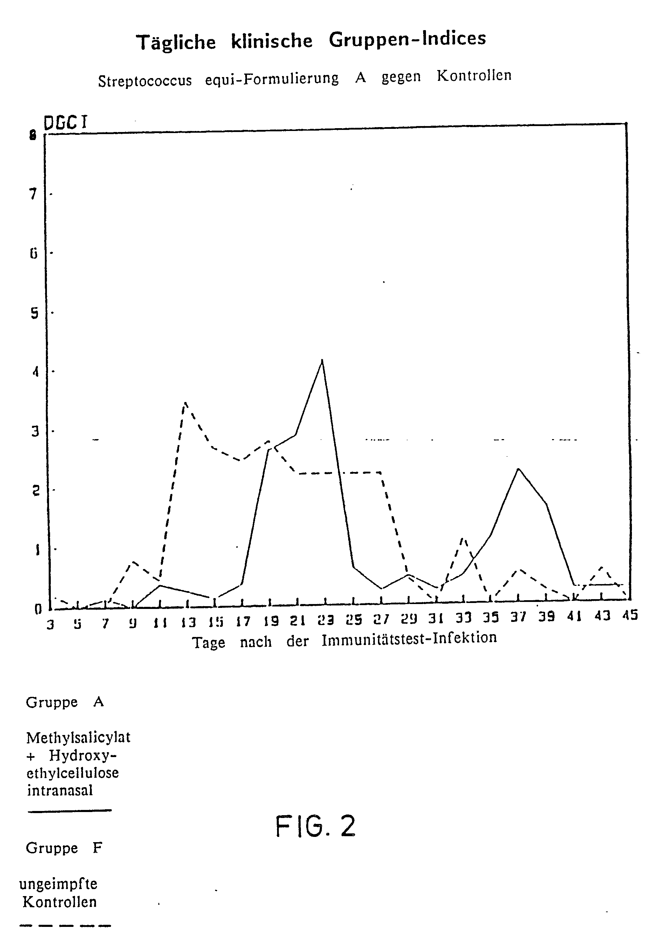

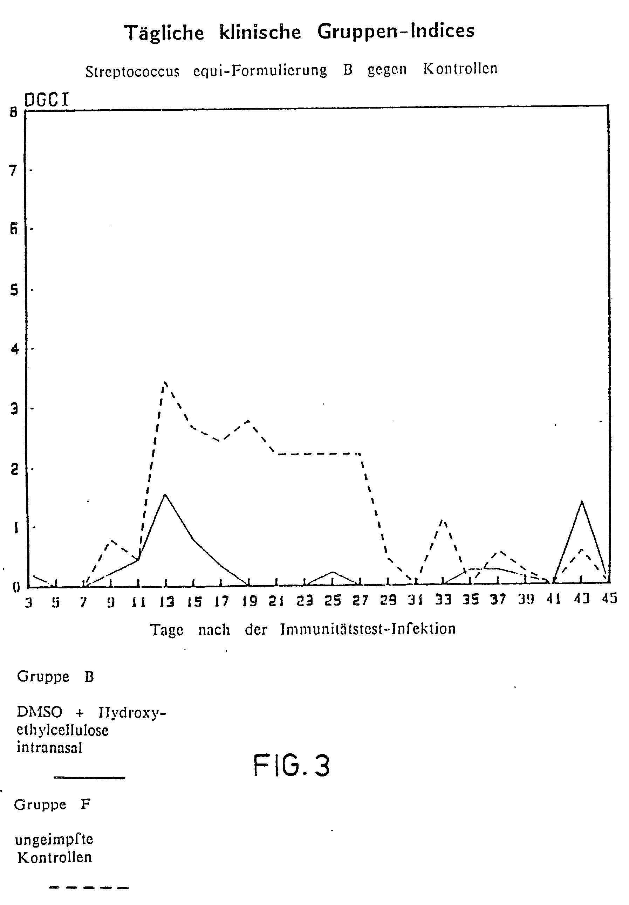

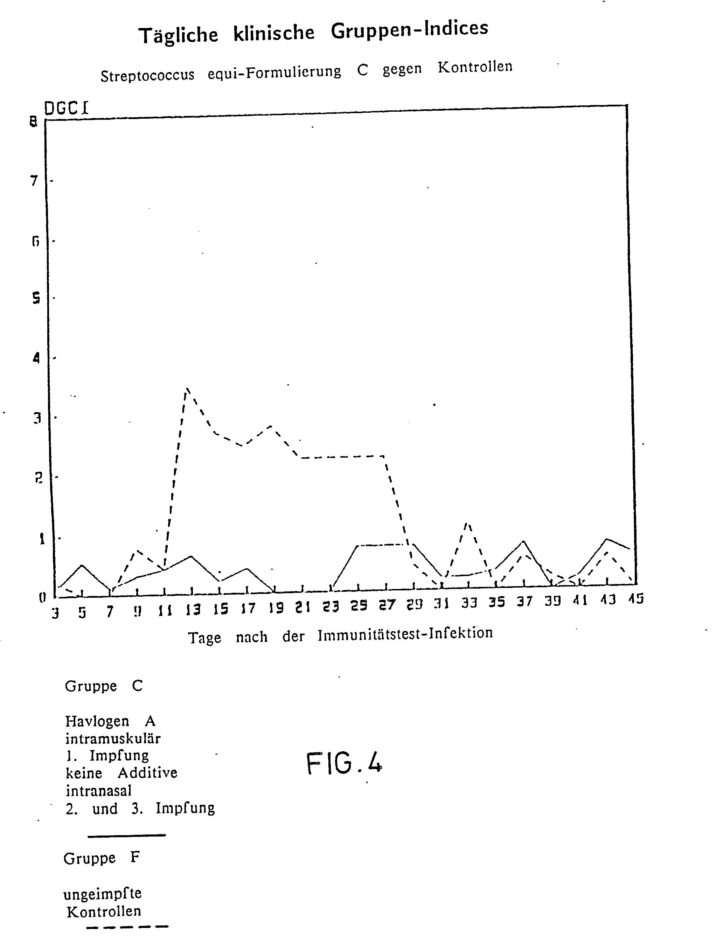

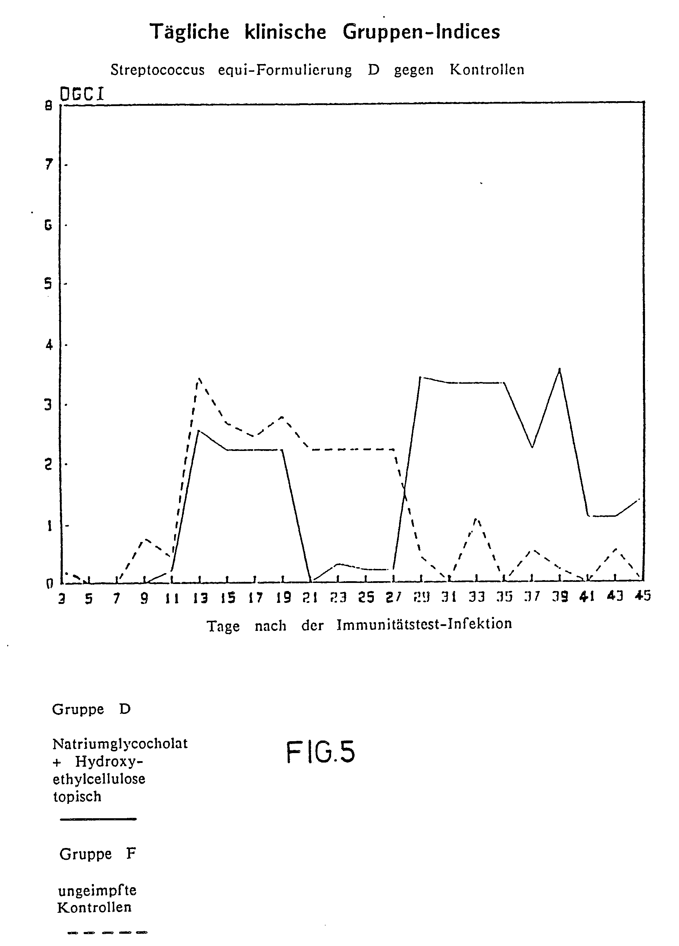

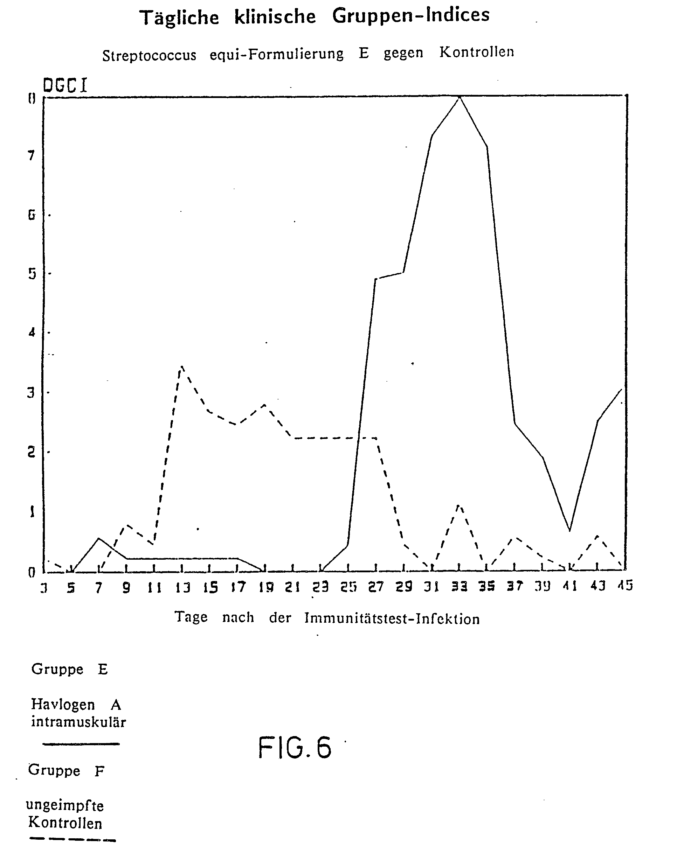

Die klinischen Beobachtungen an sechs Gruppen von Pferden, die dem Immunitätstest unterzogen worden waren, sind in Tabelle 3 und in Fig. 1 bis Fig. 6 zusammengefaßt. Tabelle 3 zeigt spezifische klinische Gruppen-Indices (SGCIs) und klinische Gesamt-Gruppen-Indices (TGCIs) für fünf Gruppen von Impflingen und eine Gruppe ungeimpfter Kontrollen. Fig. 1 zeigt ein Säulendiagramm, das die TGCIs aller Impflings-Gruppen und der Kontroll-Gruppe der Pferde vergleicht. Die Fig. 2 bis Fig. 6 vergleichen die täglichen klinischen Gruppen-Indices (DGCIs) individueller Impf-Gruppen mit denjenigen der Kontroll-Pferde.

Wie in Tabelle 3 gezeigt wird, zeigten zwei der fünf Impf-Gruppen im Vergleich mit den ungeimpften Kontrollen wenigstens eine 70-proz. Erniedrigung der klinische Gesamt-Gruppen-Indices. Die beiden wirksamsten Behandlungen waren die Behandlung B (intranasaler DMSO-Impfstoff) und die Behandlung C (Adjuvans-haltiger Impfstoff IM und nachfolgend zwei Dosierungen des kein Adjuvans enthaltenden Impfstoffes IN). Eine zusätzliche intranasale Behandlung (Behandlung A unter Verwendung von Methylsalicylat als transdermalem Träger) ergab eine Senkung des TGCI. Mit anderen Formulierungen geimpfte Pferde, eine Natriumglycocholat enthaltende topische Behandlung (Behandlung D) und eine intramuskuläre Behandlung (Behandlung E; Streptococcus equi-Antigen mit Adjuvans) zeigte höhere TGCIs als die Kontroll-Pferde. Diese letztere Behandlung (Streptococcus equi-Antigen mit Adjuvans) verzögerte jedoch den Ausbruch der Erkrankung um 14 bis 18 Tage, relativ zu den ungeimpften Kontrol-Pferden.

Die in Tabelle 3 dargestellten spezifischen klinischen Gruppen-Indices (SGCIs) zeigen, daß die Abszeß-Bildung (Lymphadenopathie) das am stärksten signifikante Zeichen ist, das bei ungeschützten Impf-Gruppen und Kontroll-Pferden auftritt.

Die Fig. 2 bis Fig. 6 die die täglichen klinischen Gruppen-Indices der geimpften Pferde mit denjenigen der Kontroll-Pferde vergleichen, zeigen die relative Wirksamkeit der Behandlungen B und C in Relation zu den weniger wirksamen Behandlungen. Diese graphischen Darstellungen zeigen, daß die beiden weniger wirksamen Behandlungen (A und E) den Ausbruch der Erkrankungen in bezug auf die Kontroll-Gruppe zu verzögern scheinen.

Die Ergebnisse der serologischen Prüdung sind in Tabelle 4 dargestellt. Diese Tabelle zeigt mittlere Titer passiver Antikörper bei Mäusen, die für die Pferde vor der Zuordnung zu den Gruppen, vor jeder Impfung und wiederum vor der Infektion für den Immunitätstest erhalten wurden. Enthalten in der Tabelle ist auch die Netto-Änderung des Antikörper-Titers (Δmitt. Titer) vom Zeitpunkt der ersten Impfung bis zum Zeitpunkt des Immunitätstests. Wie zu erwarten war, zeigen die Gruppen der intramuskulär geimpften Pferde (Gruppen C und E) relativ große der humoralen Antikörper-Spiegel (man beachte, daß Zunahmen der passiven Mäuse-Antikörper-Titer tatsächlich Abnahmen der negativen Logarithmen der Mäuse-$LD_{50}$-Werte sind). Die Gruppe E zeigte eine Zunahme des mittleren Titers von $\geq 2,0$ Logarithmus der Mäuse-$LD_{50}$. Pferde, denen die Behandlung C zuteil wurde (eine Dosis IM und nachfolgend zwei Dosen IN) zeigten ebenfalls eine beträchtliche logarithmische Zunahme des humoralen Antikörper-Titers um 1,8. Im Gegensatz dazu zeigten Pferde, die den Impfstoff intranasal oder topisch erhielten, nur eine geringfügige Zunahme des Titers, die wahrscheinlich den Test-Schwankungen zuzuschreiben ist. Da die humoralen Antikörper-Titer in vielen der am wenigsten geschützten Gruppen am höchsten waren, kann man vermuten, daß diese Titer wenig oder gar keine Beziehung zu dem tatsächlichen Schutz aufweisen.

Ein unerwartetes Ergebnis des Tests war der Tod dreier Pferde in den Gruppen B, D und E vier bis sechs Wochen nach der Infektion für den Immunitätstest. Alle drei Tiere entwickelten innere Abszesse, die an der Wand des Rektum des einzelnen Tieres hafteten. Aus den Verletzungen von zwei der drei Pferde wurde reiner S. zooepidemicus isoliert. Obwohl die Autopsie ergab, daß das dritte Pferd einen Abszeß hatte, der an der Rektalwand unmittelbar vor dem Anus haftete, wurde eine Mischung unidentifizierter Bakterien aus der Verletzung erhalten. Die Autopsie zeigte auch, daß dies Pferd anämisch war und zu wenig Gesamt-Körperfett hatte. Ein weiteres Pferd der Gruppe B entwickelte ebenfalls einen rektalen Abszeß. In diesem Fall floß jedoch der Eiter nach außen ab, und das Tier überlebte.

Nur eines der drei toten Pferde zeigte vor seinem Tod eine signifikante klinische Antwortreaktion auf den Immunitätstest (d.h. Nasenausfluß oder Abszeß-Bildung). Zum Zwecke der Daten-Auswertung wurden die klinischen Zeichen der drei Pferde bis zum Tage ihres Ablebens in die Gruppen-Analysen (DGCI, TGCI) einbezogen. Da die Todesfälle von einem Faktor resultierten, der nicht unmittelbar im Zusammenhang mit der Infektion durch S. equi stand, wurden keine zusätzlichen klinischen Index-Punkte vergeben.

Tabelle 3

| Pferde-Immunitätstest gegen Streptococcus equi: Sechs intranasale, intramuskuläre, topische Formulierungen | | | | | | | | |
|---|---|---|---|---|---|---|---|---|
| Klinische Antwortreaktion der Immunitätstest-Gruppen (Spezifische klinische Gruppen-Indices und klinischer Gesamt-Gruppen-Index) | | | | | | | | |
| Gruppe | Beschreibung | Dosis/Weg | Spezif. klin. Gruppen-Indices | | | | klin. Gesamt-Grupp.-(TGCI) | Prozent Vermind. d. TGCI gegen Kontrol. |
| | | | Abszeß-Bildung (SGCI ) | Nasen-Ausfluß (SGCI ) | Temp.-Anstieg (SGCI ) | Zunahme d.weiß. Blutkörp. (SGCI ) | | |
| A (n = 8) | Methylsalicylat plus HEC* | 3 intranasal (2,0 ml) | 5,0 | 3,1 | 3,9 | 4,5 | 16,5 | 34,3 % |
| B (n = 9) | DMSO plus HEC* | 3 intranasal (2,0 ml) | 0 | 1,1 | 3,0 | 1,7 | 5,8 | 76,9 % |
| C (n = 9) | Adjuvans keine Additive | 1 intramuskulär 2 intranasal (2,0 ml) | 0 | 1,7 | 2,4 | 2,0 | 6,1 | 75,7 % |
| D (n = 9) | Natriumglycocholat plus HEC* | 3 topisch (2,0 ml) | 24,4 | 10,0 | 2,0 | 0 | 36,4 | 0 % |
| E (n = 9) | Adjuvans | 2 intramuskulär (2,0 ml) | 31,5 | 5,0 | 3,7 | 3,8 | 43,6 | 0 % |
| F (n = 9) | ungeimpfte Kontrollen | entfällt | 17,8 | 3,3 | 2,4 | 1,6 | 25,1 | entfällt |

* HEC = Hydroxyethylcellulose

EP 0 304 786 A2

Tabelle 4

| Pferde-Immunitätstest gegen Streptococcus equi: Sechs intranasale, intramuskuläre, topische Formulierungen | | | | | | | | |
|---|---|---|---|---|---|---|---|---|
| Gruppen-serologische Antwortreaktion: Mittlere Titer passiver Antikörper bei Mäusen* | | | | | | | | |
| Gruppe | Beschreibung | Dosis/Weg | vor Reihen Untersuch. | vor Impfung | vor Auffrisch-Impfung | vor 2. Auffr.-Impfung | vor dem Immun-Test | mittl. Titer** |
| A (n = 8) | Methylsalicylat plus HEC*** | 3 intranasal (2,0 ml) | 5,9 | 5,5 | 5,4 | 5,2 | 5,2 | 0,3 |
| B (n = 9) | DMSO plus HEC*** | 3 intranasal (2,0 ml) | 5,9 | 6,1 | 6,1 | 6,0 | 5,8 | 0,3 |
| C (n = 9) | Adjuvans keine Additive | 1 intramuskulär 2 intranasal (2,0 ml) | 6,0 | 6,6 | 4,2 | 5,0 | 4,8 | 1,8 |
| D (n = 9) | Natrium glycocholat plus HEC*** | 3 topisch (2,0 ml) | 6,0 | 5,9 | 5,7 | 6,1 | 5,4 | 0,5 |
| E (n = 9) | Adjuvans | 2 intramuskulär (2,0 ml) | 6,0 | 6,8 | 4,4 | entfällt | 3,6 | 3,2 |
| F (n = 9) | ungeimpfte Kontrollen | entfällt | 5,9 | 5,4 | 5,8 | entfällt | 4,7 | 0,7 |

* Der mittlere Titer ist der Mittelwert der Logarithmen der $LD_{50}$ bei Mäusen, erhalten bei Prüfung von Pferde-Sera mittels des in der US-PS 4 529 582 beschriebenen passiven Mäuse-Schutz-Tests.

** mittl. Titer ist die zahlenmäßige Differenz zwischen den Titern vor dem Immunitäts-Test und vor der Impfung.

*** HEC = Hydroxyethylcellulose mit einer Viskosität einer 2-proz. Lösung bei Raumtemperatur von 4500 bis 6500 mPa.s (cP).

EP 0 304 786 A2

## H. Auswertung

Die Ergebnisse dieses Versuchs zeigen an, daß ein intranasal verabreichter Bakterien-Extrakt von Streptococcus equi Pferde in einem experimentellen Immunitätstest gegen Infektion zu schützen vermag. Die Behandlung mit drei Dosierungen IN mit der DMSO enthaltenden Formulierung reduzierte im Vergleich zu ungeimpften Kontroll-Pferden die klinischen Zeichen um insgeamt 76,9 %. Ebenfalls besonders wirksam in diesem Test erschien eine Verordnung von drei Dosierungen, bestehend aus einer Dosis eines Adjuvans-haltigen Impfstoffes IM und zwei nachfolgend IN verabreichten Dosen eines nicht Adjuvans-haltigen Antigen-Extrakts.

Obwohl bei den zwei vorher beschriebenen Formulierungen stark positive Ergebnisse vermerkt wurden, wurden unerwartet schlechte Ergebnisse bei der Behandlung E boebachtet, die für das gegenwärtig vom USDA [U.S. Department of Agriculture] zugelassene 2 ml-STREPGUARD-Produkt repräsentativ ist. Diese Impfstoff-Formulierung hatte vorher ihre Wirksamkeit in einer Immunitätstest-Untersuchung an 59 Pferden erwiesen. Ein Grund für die differierenden Ergebnisse könnte mit dem Verfahren des Immunitätstest zusammenhängen. In der früheren Untersuchung war die Immunitätstest-Infektion weitgehend indirekt (die Pferde wurden in ein endemisches Gebiet verbracht, und virulenter S. equi wurde nur in das Maul eines jeden Pferdes eingebracht). In dem laufenden Versuch wurden die Organismen von S. equi direkt in die Nasenwege injiziert. Es ist möglich, daß die zweite Methode der Immunitätstest-Infektion das intramuskuläre Produkt zu "uberwältigen" vermag. Die Tatsache, daß die Behandlung E den Ausbruch der Krankheit bei geimpften Pferden verzögerte, könnte diesen Schluß indirekt stützen. Man würde nicht erwarten, daß der intramuskuläre Impfstoff eine starke lokale sekretorische Antwortreaktion stimuliert. Unter den Gegebenheiten kann das vorliegende Ergebnis den Beweis für die Notwendigkeit der intranasalen Verabreichung eines Produkts liefern, um lokales IgA zu stimulieren und für eine stärkere lokale Immunität zu sorgen.

Die Tatsache, daß humorale Antikörper-Titer, wie sie im Mäuse-Test auf passive Antikörper gemessen werden, nicht im Zusammenhang mit der Schutzwirkung stehen, ist nicht besonders überraschend. Ein ähnliches Ergebnis wurde in einem früheren Infektions-Test mit einem Bakterien-Extrakt von S. equi beobachtet. Es erscheint wahrscheinlich, daß der Schutz gegen eine experimentelle Infektion durch S. equi einen lokalen Mechanismus einschließt, der auf der Ebene der Nasenschleimhaut arbeitet.

## Ansprüche

1. Verfahren zur Herstellung von Impfstoffen zur Immunisierung von Pferden gegen mikrobiologische Infektion durch Aufbringen inaktivierter Organismen, antigener Extrakte oder rekombinanter DNA oder synthetischer Peptid-Antigene der invasiven Species auf die Nasenschleimhaut oder das tonsilläre Gewebe von Pferden.

2. Verfahren nach Anspruch 1, dadurch gekennzeichnet, daß das antigene Material in einen flüssigen Träger eingearbeitet wird und als Flüssigkeitsstrom oder in Form großer Tröpfchen auf die Nasenwand in der Nähe des tonsillären Gewebes gesprüht wird.

3. Verfahren nach Anspruch 1, dadurch gekennzeichnet, daß der infizierende Mikroorganismus ein solcher ist, der eine Erkrankung der Atemwege des Pferdes verursacht.

4. Verfahren nach Anspruch 3, dadurch gekennzeichnet, daß der Mikroorganismus, gegen den Schutz erzielt werden soll, Streptococcus equisimiles, Streptococcus zooepidemicus oder Streptococcus equi ist.

5. Verfahren nach Anspruch 1, dadurch gekennzeichnet, daß der infizierende Mikroorganismus das Pferdeinfluenza-Virus ist.

6. Verfahren nach Anspruch 1, dadurch gekennzeichnet, daß eine Kombination aus abgetötetem Pferdeinfluenza-Virus und einem antigenen Extrakt aus Streptococcus zooepidemicus oder Streptococcus equi auf das tonsilläre Gewebe zur Einwirkung gebracht wird.

7. Verfahren nach Anspruch 2, dadurch gekennzeichnet, daß der flüssige Träger auch ein Eindickungsmittel enthält.

8. Verfahren nach Anspruch 7, dadurch gekennzeichnet, daß das Eindickungsmittel auf Cellulose oder Hydroxyethylcellulose basiert.

9. Verfahren nach Anspruch 2, dadurch gekennzeichnet, daß der flüssige Träger auch ein die Haut durchdringendes Mittel enthält.

10. Verfahren nach Anspruch 9, dadurch gekennzeichnet, daß das die Haut durchdringende Mittel entweder Dimethylsulfoxid oder Methylsalicylat ist.

# Klinische Gesamt-Gruppen-Indices

für die Gruppen der Immunitätstest-Infektion
mit Streptococcus equi

FIG.1

## Tägliche klinische Gruppen-Indices

Streptococcus equi-Formulierung A gegen Kontrollen

Tage nach der Immunitätstest-Infektion

Gruppe A

Methylsalicylat
+ Hydroxyethylcellulose
intranasal

_____

Gruppe F

ungeimpfte
Kontrollen

- - - - - -

FIG. 2

# Tägliche klinische Gruppen-Indices

Streptococcus equi-Formulierung B gegen Kontrollen

Tage nach der Immunitätstest-Infektion

Gruppe B

DMSO + Hydroxyethylcellulose
intranasal

_____

Gruppe F

ungeimpfte
Kontrollen

- - - - -

FIG. 3

# Tägliche klinische Gruppen-Indices

Streptococcus equi-Formulierung C gegen Kontrollen

Tage nach der Immunitätstest-Infektion

Gruppe C

Havlogen A
intramuskulär
1. Impfung
keine Additive
intranasal
2. und 3. Impfung

FIG.4

Gruppe F

ungeimpfte
Kontrollen

## Tägliche klinische Gruppen-Indices

Streptococcus equi-Formulierung D gegen Kontrollen

Tage nach der Immunitätstest-Infektion

Gruppe D

Natriumglycocholat
+ Hydroxyethylcellulose
topisch

FIG.5

Gruppe F

ungeimpfte
Kontrollen

## Tägliche klinische Gruppen-Indices

Streptococcus equi-Formulierung E gegen Kontrollen

Tage nach der Immunitätstest-Infektion

Gruppe E

Havlogen A
intramuskulär

————————

Gruppe F

ungeimpfte
Kontrollen

— — — — — —

FIG. 6